# EUROPEAN PATENT SPECIFICATION

(11) **EP 1 130 995 B1**
(45) Date of publication and mention of the grant of the patent: **25.01.2006**
(21) Application number: 99957743.0
(22) Date of filing: 18.11.1999
(51) Int. Cl.: A61B 3/12, G01B 9/02

(54) **LIMITED COHERENCE STEREO OPHTHALMOSCOPE**
STEREO-OPHTALMOSKOP MIT BEGRENZTER KOHÄRENZ
STEREO-OPHTALMOSCOPE

(30) Priority: 18.11.1998 AU PP718598
(43) Date of publication of application: 12.09.2001
(73) Proprietor: Lions Eye Institute Limited, Nedlands, W.A. 6009 (AU)
(72) Inventor: VAN SAARLOOS, Paul, Phillip, Karrinyup, W.A. 6018 (AU); REINHOLZ, Fred, Norbert, Floreat, W.A. 6014 (AU)
(74) Representative: Piésold, Alexander James
(86) International application number: PCT/AU1999/001024
(87) International publication number: WO 2000/028885

(56) References cited:
- EP-A- 0 509 903
- US-A- 5 537 162
- US-A- 5 633 694
- US-A- 5 784 148
- US-A- 5 975 697
- BAUMGARTNER ANGELA ET AL: "Signal and resolution enhancements in dual beam optical coherence tomography of the human eye" J BIOMED OPT;JOURNAL OF BIOMEDICAL OPTICS JAN 1998 SOCIETY OF PHOTO-OPTICAL INSTRUMENTATION ENGINEERS, BELLINGHAM, WA, USA, vol. 3, no. 1, January 1998 (1998-01), pages 45-54, XP002278208
- DREXLER W ET AL: "MEASUREMENT OF THE THICKNESS OF FUNDUS LAYERS BY PARTIAL COHERENCE TOMOGRAPHY" OPTICAL ENGINEERING, SOC. OF PHOTO-OPTICAL INSTRUMENTATION ENGINEERS. BELLINGHAM, US, vol. 34, no. 3, 1 March 1995 (1995-03-01), pages 701-710, XP000495215 ISSN: 0091-3286

## Description

### Field of the Invention

The present invention relates to a method and apparatus for the stereoscopic examination of, for example, the fundus of the eye, with applications in the investigation and diagnosis of diseases that affect the posterior chamber of the eyeball. The invention will be described by reference to such applications, but it is envisioned that the apparatus and method of the present invention may be used for stereoscopic imaging in other medical processes.

### Background Art

Visualisation of the ocular fundus can provide important information about the state of the eye and of the body. Information concerning ocular and systemic diseases, such as glaucoma, macula degeneration, diabetes or hypertension can be gained from examination of the posterior pole of the eye. In the past, imaging of the ocular fundus has been performed by means of an ophthalmoscope, with which a direct view of the retina can be obtained. Other methods include the use of fundus cameras to obtain photographic images. However, these techniques usually require the use of mydriatic dilating drugs. The amount of light required to illuminate the fundus may also be uncomfortable for the patient.

Recent developments have resulted in the emergence of a new imaging instrument for the ophthalmologist, in which an image of the eye may be observed in real-time and captured on a television monitor or screen, during procedures such as fluorescein angiography. This instrument, known as a Scanning Laser Ophthalmoscope (SLO), first described in US Patent 4,213,678, is currently used to produce representations of the ocular fundus in two dimensions. US Patents 4,765,730, 5,268,711 and 5,430,509 describe different embodiments of the scanning laser ophthalmoscope. All utilise a laser beam or light source that is directed through the pupil and onto the retina by way of two-directional, scanning mechanisms. Light from the laser is reflected off the retinal wall towards a photosensitive detection device. Electro-optical circuitry is employed to convert the light into synchronized signals, so that it is possible to display an image of the fundus on a television screen or monitor.

However, although the optic disc region and retinal layers have a three dimensional structure, the existing SLO technology described above does not permit stereoscopic viewing of the ocular fundus. Stereoscopic images of the ocular fundus can impart valuable information that cannot otherwise be derived from a two dimensional representation, especially in relation to the diagnosis of glaucoma. Efforts have therefore been made to create a device capable of producing three dimensional fundus images, while improving on the contrast and resolution of conventional SLO images.

Frambach, Dacey and Sadun (1992, 1993) describe a method of producing a three dimensional fundus picture during fluorescein angiography, using a modified SLO. To obtain stereoscopic data the SLO was manually moved from side to side during angiogram proceedings, much like a fundus camera is moved to enable viewing from two different positions individual frames from the video tape were chosen from left and right perspectives to provide a three dimensional image. An alternative approach employed by the Frambach et al. involved the use of a modified Allen separator. A piece of flat glass was attached to an extended rod, which was coupled to the Alien separator, so that the glass was interposed between the eye and the SLO. The glass was then rapidly rotated to provide the left and right perspectives. The resulting frames were digitized by computer and viewed directly on a video screen. Superimposed images were formed by breaking a stereo pair down into corresponding fields and recombining them to form a single frame. LCD glasses were then used to view the left and right fields with the corresponding eye in turn.

These disclosures illustrate that achieving a stereoscopic image from a conventional scanning laser ophthalmoscope is possible. However, the methods involved exhibit a number of disadvantages. Frambach et al.'s first method of shifting the SLO involved awkward and confusing adjustments, resulting in poor stereoscopic image quality. The second method also had the disadvantage that interference due to unwanted back reflections from the Allen separator would hinder the transmission of stereoscopic information. Unrequired scattered light would impinge on the photodetecting element, causing a decrease in the contrast and resolution of the images.

Improvements in SLO image resolution and contrast are possible if the detector receives light only from the plane of interest and not scattered light from the media of the eye. A scanning laser ophthalmoscope that could provide high resolution, high contrast images of an ocular fundus was realised with the invention of the confocal scanning laser ophthalmoscope (cSLO), such as that described in US Patents 5,170,276 and 5,071,246. The confocal SLO utilises a pinhole or slit aperture to focus the light reflected from the fundus onto a photodetecting element. The aperture is located at a plane in which the opening is conjugate with the plane of the fundus of the eye. In this way, only the light reflected from the plane of interest impinges on the photodetecting element and any light scattered or reflected from out-of-focus planes is prevented from degrading the image.

Confocal scanning laser ophthalmoscopes are also currently used to provide three dimensional information concerning the ocular fundus. The confocal aperture of the cSLO allows the user to focus precisely on specific layers of the retina. By adjusting the focal plane of the aperture, images can be captured at different levels in the fundus, to reproduce desired depth characteristics. In this way a number of "optical sections" can be produced. A computer can then be used to extract depth information, through the process of "stacking" a selection of the optical sections taken at different levels of the retina. Information regarding the third dimension can therefore be interpolated.

US Patent No. 4,900,144 (also see Optics Communications: 87(1,2): 9-14) describes a scanning laser ophthalmoscope that employs an alternative confocal focussing arrangement. The invention can produce a three dimensional representation of an object that displays multiple reflectivity characteristics (such as the ocular fundus) through a method slightly different from the conventional confocal depth production methods described above. This US patent teaches the use of two separate confocal slit apertures and photodetecting units. The detection slits are orientated parallel to the direction in which the light, reflected from the fundus, is scanned. However, both slits are slightly displaced from the normal position: the apertures are not conjugate with the fundus of the eye. One is positioned slightly forward of the conjugate plane, while the other is placed to the rear. Owing to the positioning of the confocal apertures, the output signals from the photodetectors vary in intensity according to the unevenness of the fundus. The resulting output signals are processed electronically by division calculations, detailed in US Patent No 4,900,144, to obtain a three dimensional profile. The resultant real-time image displays the topography of the fundus through different shade levels, reflecting different retinal depth levels. Software may also be used to create three dimensional graphic patterns.

The methods described above use the depth discrimination, or axial resolution, of the confocal system. Unlike lateral resolution, axial resolution is strongly limited by two factors. Firstly, the shape and size of the laser light focus which is scanned over the retina may suffer from deformations and distortions, particularly in the direction of the optical axis. This is due to the limited useful numerical aperture of the eye and its optical imperfections. Furthermore, the axial resolution of a confocal SLO may be constrained by the size of the detection pinhole or slit. Owing to intensity limitations on the living eye it is often necessary to provide a detector aperture size that is larger than the optimal confocal pinhole in order to maintain a sufficient signal to noise ratio. Owing to these two factors, the axial resolution of a confocal system is typically thirty times less than the lateral resolution.

A further technique to produce three dimensional images of the ocular fundus, known as scanning laser triangulation, is described by Milbocker and Reznichenko (1991). Triangulation is a method commonly used for measuring distances. Combined with a confocal aperture, this method involves synchronized scanning of a pixel of light across the fundus by way of two mirrors. The illumination and detection paths are arranged symmetrically and are defined by the two mirrors. The axial distance is measured by the displacement of the illuminated spot in the confocal plane, enabling calculation of the depth from the points above and below the average, position of the retinal wall. With this method the axial resolution is directly coupled to the lateral resolution. However, owing to the pupil size of the human eye the useful triangulation angles are small. Consequently, the axial resolution is about 10 times worse than the lateral resolution.

Interferometry is an optical method that is much better suited for distance measurements since it does not depend on the focussing or imaging qualities of optical elements as do, for example, the cornea and the lens of the eye. Interference patterns can only occur when the difference between the length of the reference arm and the object arm is shorter than the coherence length of the light source. Non-laser light sources such as bulbs, LEDs or superluminescent diodes have a coherence length of only a few micrometres. Thus, the detection of interference patterns means that the object distance is equal to the reference distance determined with an accuracy equal to the coherence length.

Popoleanu *et al.* (*Journal of Biomedical Optics,* Jan. 1998, vol. 3, no. 1, p. 12-20) describe an apparatus suited for transversal and longitudinal imaging of the retina using low coherence reflectometry. The light in the object arm of a fibre-based interferometer, where a superluminescent diode is the light source, propagates through a phase modulator and is scanned over the retina in a raster pattern. The light reflected from the fundus of the eye is combined with the light in the reference arm of the interferometer which length is controlled by means of moveable mirrors. Frequency sensitive detection recognizes the occurrence of interference. The strength of the interference signal is used by a frame grabber to form an image of features that are situated in a thin layer in the back of the eye. The axial position of this layer is controlled by the movable mirrors in the reference arm while the thickness of the layer is determined by the coherence length of the light source. In much the same way as with a confocal scanning laser ophthalmoscope a three dimensional image can be computed from a number of optical sections (transversal images) at different axial positions. Alternatively, scans in only one lateral and the axial direction (x-z-scans) may be carried out to record longitudinal images. The images acquired with this apparatus have a high depth resolution, about ten times better than a confocal scanning laser ophthalmoscope. However, the length of the object arm of the interferometer varies with the scan and/or the focal position as well as with the position and the size of the individual eye which is examined. It is therefore impractical or impossible to obtain quantitative data for the measurement of absolute distances in the eye ball. Furthermore, with the described lay-out a reduction in recording time is difficult due to bandwidth limitations of the phase modulation and the speed of the galvo-scanners.

A dual beam system as described by Baumgartner *et al.* (*Journal of Biomedical Optics,* Jan. 1998, vol. 3, no. 1, 45-54) overcomes one of the aforesaid problems. An external interferometer produces a beam with two coaxial components which have a path difference of twice the difference in the arm length of the interferometer. This beam is guided onto the eye, where parts of the beam are reflected from the cornea - which acts as a reference surface - and other parts are reflected from the fundus of the eye. If the optical distance between the cornea and a certain fundus feature matches the difference in length between the interferometer arms (within the accuracy of the coherence length of the light source) interference signals are detected. It is then straightforward to get a readout for the distance in the eye ball from the positions of the two interferometer mirrors. In order to achieve good signal-to-noise ratios for the interference detection a sub-component of the illumination beam has to be focused on the cornea while the rest of the beam has to enter the pupil of the eye in a more or less collimated state. In this configuration the focused component travels through air only whereas the collimated component has to travel through the dispersion causing ocular medium also. It is demonstrated that a dispersion compensating element can therefore be used advantageously and an axial resolution of only a few micrometres can be achieved. To split the illuminating beam into a focused and a collimated component Baumgartner et al. use a special Fresnel lens-like diffractive optical element which is placed in front of the eye. However, the use of this non-standard element can cause unwanted back reflections and transmission losses. Lateral scanning of the beam, which is necessary to acquire data not only for one point but for a line or an area of the retina, is also severely restricted.

In order to detect the occurrence of interference a time variation in the interference pattern is necessary. In this lay-out the Doppler-shift caused by the moving reference mirror creates this time variation, it is therefore not possible to record a limited coherence image of a layer of the retina for a fixed axial position of this layer.

Accordingly, there remains a need to provide apparatus able to be adapted, at least in an embodiment, as a limited coherence scanning ophthalmoscope capable of acquiring images with a high depth resolution and of quantifying the 3D-morphology of the back of the eye, which preferably is not restricted by any of the aforementioned limitations.

It is therefore an object of the present invention, at least in one or more preferred embodiments, to provide an improved method and apparatus for producing a high contrast, three dimensional representation of a scanned object, based on both the high lateral resolution of the reflection characteristics of that object and the high axial resolution of the limited coherence reflectometry.

It is a further object of the present invention, at least in an advantageous application, to achieve the above object with a novel limited coherence scanning ophthalmoscope design that incorporates the use of additional beam splitting, focussing and beam combining components to gain information and quantitative topographic data about the third dimension.

### Summary of the Invention

In a first aspect, the invention provides an ophthalmoscope for the three dimensional imaging of an eye including:
first beam modifying means for modifying an incident beam of short coherence length light to form a modified beam of first and second components having a mutual path difference and being capable of producing a detectable interference;
beamsplitting means for splitting paid modified beam into first and second beams;
second beam modifying means for changing the divergence of at least one of said first and second beams;
recombining means for thereafter recombining said first and second beams;
means for directing said recombined first and second beams towards a surface of said eye and scanning them across the surface; and
means for monitoring the first and second beams after reflection and detecting interference of the reflected first and second beams.

Preferably, in its first aspect, the invention further includes steering means to vary a nodal point of the scanned first and second beams.

Preferably, the first beam after said changing of the divergence of at least one of the first and second beams is focused on a position in front of the surface for reflection at said position. The second beam may be a collimated beam at the scanning means, for being focussed onto the surface.

Advantageously, the scanning means is arranged for scanning in at least two dimensions.

In an advantageous application, the apparatus is for imaging and/or measuring a surface comprising an ocular fundus, e.g. the incident beam is a laser beam, and the apparatus functions in use as a scanning laser ophthalmoscope. In this case, the first and second beams may respectively be a focussed beam arranged to be at least partially reflected from the cornea of an eye, and a collimated beam for being focussed by the eye onto its fundus for reflection thereby. In a second aspect, the invention provides a method for the three dimensional imaging of an eye including:
modifying an incident beam of short coherence length light to form a modified beam of first and second components having a mutual path difference and being capable of producing a detectable interference;
splitting said modified beam into first and second beams;
changing the divergence of at least one of said fiat and second beams and thereafter recombining said first and second beams;
directing said recombined first and second beams towards a surface of said eye and scanning them across the surface, and
monitoring the first and second beams after reflection and detecting interference of the reflected first and second beams.

The invention further provides, in a third aspect, method of measuring the distance of a point D on a surface of the eye to a plane AB using an ophthalmoscope as described in claim 1, the ophthalmoscope including a beam steering means for directing the first and second components from point A and remote point B, the method comprising the following steps:
obtaining a first set of images of the surface of the eye, taken at a certain retinal depth from point A, at least one image in the first set of images including point D;
obtaining a second set of images of the surface of the eye, taken at a certain retinal depth from point B, at least one image in the second set of images including point D;
determining the distance AD from the path difference between the first and second beams of the ophthalmoscope associated with an image including point D in the first set of images;
determining the distance BD from the path difference between the first and second beams of the ophthalmoscope associated with an image including point D in the second set of images;
obtaining the distance AB from the position of predetermined optical elements of the ophthalmoscope; and
calculating the perpendicular distance from plane AB to point D using triangulation methods.

Preferred, advantageous and optional features of the apparatus as described above ate where appropriate also applicable as preferred, advantageous or optional steps of the method of the second or third aspect of the invention.

### Brief Description of the Drawings

In order that the invention be more fully ascertained, some preferred embodiments will be described, by way of example, with reference to the accompanying drawings, in which:
Figure 1 is a schematic diagram of a first preferred embodiment of the present invention; and
Figure 2 illustrates the process of data and image acquisition.

### Preferred Embodiments

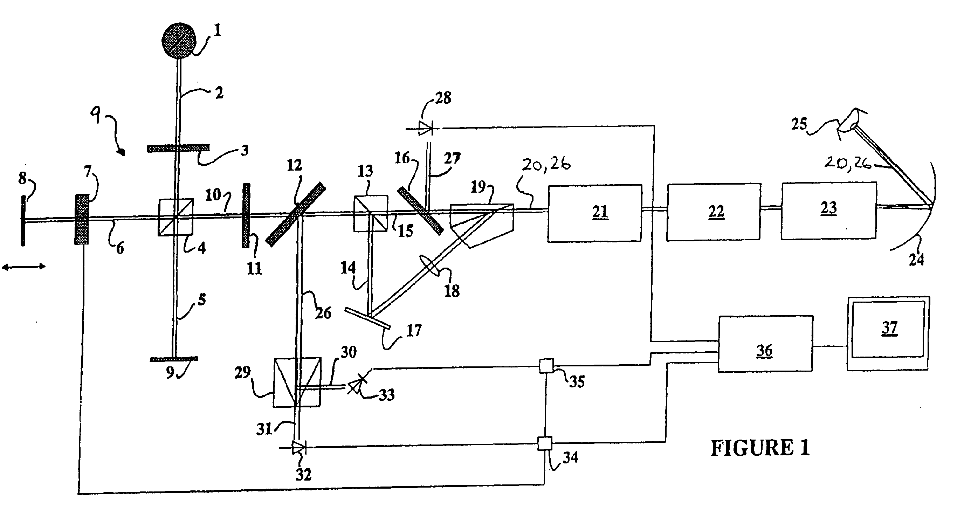

Refening to Figure 1, a preferred embodiment of the present invention utilises a superluminescent diode source 1, or any other suitable source of light with a short coherence length including collimating optics, which emits a light beam 2. Alternatively, two or more light sources may be utilised to produce beam 2. This beam is directed through a polariser 3 onto a first beamsplitter 4 which forms part of an interferometric set-up 9. Components 5 and 6 of beam 2 are impinged onto mirrors 8 and 9 respectively, and reflected back to first beamsplitter 4. Mirror 8 is movable along the axial direction and the position of mirror 8 is computer controlled. Component 6 passes through the high fixed frequency phase modulator 7 which continuously changes the phase but not the intensity or the polarisation state of component 6. Alternatively, the Doppler effect may be used to create a frequency shift in component 6 when moving the mirror 8.

Beamsplitter 4 recombines components 5 and 6 to form beam 10 which consists of two sub-components with a difference in path length determined by the positions of mirrors 8 and 9. The beam 10 passes through half-wave plate 11, which rotates the polarisation direction of the linearly polarised beam 10 and determines the relative intensities of the components 14 and 15. Alternatively, a variable retarder in conjunction with a quarter wave plate can replace half wave plate 11.

Beam 10 is then guided (through beamsplitter 12-see below) onto a second beamsplitter in the form of polarizing beamsplitter 13, which produces the two components, ie first and second beams 14 and 15 with perpendicular polarisation directions. Component 14 is impinged off mirror 17 towards lens 18 to produce a non-collimated beam in such a way that component 14 will finally be focused on the cornea of the eye 25. Alternatively, a combination of lenses and/or flat and/or curved mirrors can replace lens 18. Component 15 passes through beamsplitter 16, and a portion of component 15 reaches the beamsplitter 19, preferably a Thompson prism. Beamsplitter 19 re-combines components 14 and 15 to form beam 20. As mentioned, component 14 is focused on the cornea of eye 25, whereas component 15 is collimated.

The beam 20 is directed towards the focussing unit 21, consisting of a lens or combinations of lenses and mirrors.

After leaving the focussing unit 21 the beam is guided through the scanning unit 22 where a scan pattern is produced to image areas of interest of the eye. Within scanning unit 22 a beam may be guided onto a resonant scanner or a rapidly rotating multiple facet mirror, which acts as a horizontal scanner, and subsequently onto a small curved mirror, which shapes the beam into a horizontal line. The beam may then travel to a vertical scanner such as a galvanometer controlled mirror.

After leaving the scanning unit 22 the beam 20 passes through a beam steerer in the form of beam steering unit 23, which allows for moving the nodal point of the scan pattern to different positions within the pupil. Preferably, the beam steering unit 23 consists of a pair of toggling mirrors. These mirrors are positioned so that each directs the beam 20 onto the surface to be imaged from two slightly different directions and positions, with substantially overlapping imaging areas. They preferably toggle every alternate frame, such that visual information is received from the right and left perspectives in alternate frames. Alternatively, the two toggling mirrors could be substituted with a single mirror that can change position preferably every second frame or half frame, or a galvanometer mounted prism or glass plate that is capable of imaging from the left and right perspective every half frame.

After leaving the beam steering unit 23 the light is reflected off a large curved mirror 24 before entering the eye 25. Component 14 of the beam 20 is focussed onto the cornea and is reflected off it to provide a reference signal. Component 15 of beam 20 enters the eye through the pupil and passes through the eye's internal structure to reach the retina at the back of the fundus. The light is reflected off the retinal layers and exits through the pupil.

It will be appreciated that by forming component beams 14, 15 before scanner 22, the scanner is able to scan the recombined beam, thus enhancing flexibility in lateral scanning. The prior complex diffractive element in front of the eye is also avoided.

The reflected beam 26, including reflections of incident components 14, 15, traverses the same output path as the incident beam 20. Beamsplitter 19 divides beam 26 into a component that is reflected from the cornea and a component reflected from the fundus. The latter component reaches the non-polarising beamsplitter 16 where parts of the light reflected from the fundus form beam 27. The intensity of beam 27 is measured by photodetector 28, preferably a photomultiplier tube or an avalanche photodiode, to form the standard scanning ophthalmoscopic image of the fundus of the eye. Alternatively, focussing optics and/or an aperture may be placed in front of the photodetector, or optional photodetector 32 and/or 33 may be used to detect the standard scanning ophthalmoscopic image which is offset by the reflected intensity of the cornea. Alternatively, half wave plate 11 may be adjusted so that component 14 vanishes and photodetector 32 and/or 33 detects the standard scanning ophthalmoscopic image. Beamsplitter 13 re-combines the light which passes through beamsplitter 16 with light which leaves beamsplitter 19, passes through lens 18 and is reflected off mirror 17 to reform beam 26.

Upon reaching the third beamsplitter in the form of nonpolarizing beamsplitter 12, part of the light is reflected by the beamsplitter towards the polarising beamsplitter 29 which is rotated by 45°. Both the beams 30 and 31, which are polarised perpendicular to each other, contain reflected light from both the cornea and the fundus of the eye 25. Interference in both the beams 31 and 32 will occur when the path difference of the light reflected from the cornea and the light reflected from the fundus equals the optical distance between the cornea and the fundus within the tolerances of the coherence length of the light source 1. Since the path difference of the two subcomponents of beams 10 and 20 is set by the positions of the mirrors 8 and 9, the distance from the fundus feature which is imaged to the cornea can be measured by determining the positions of the mirrors 8 and 9 when interference is established.

The intensities of the beams 30 and 31 are detected using the photodetectors 32 and 33, by preference photomultiplier tubes or avalanche photodiodes. Alternatively, focussing optics and/or apertures may be placed in front of the photodetectors. Signal processing means 34 and 35 compare the signals from the photodetectors 32 and 33 with the frequency of the phase modulator 7 to recognise interference patterns. Alternatively, the signals of the photodetectors 32 and 33 can be compared to a frequency resulting from the Doppler shift when moving mirror 8. Alternatively, the signals of the photodetectors 32 and 33 may be subtracted from each other to reveal interference patterns.

The outputs of the photodetector 28 and the signal processing means 34 and 35 are then sent to the computer 36. An imaging board in computer 36 and appropriate electronic hardware and software convert these outputs and information on the position of the mirror 8 into images, including three dimensional topological representations of the fundus of the eye. The images are displayed by computer monitor 37. It is then possible to calculate three dimensional distances between features chosen by the computer operator from those images.

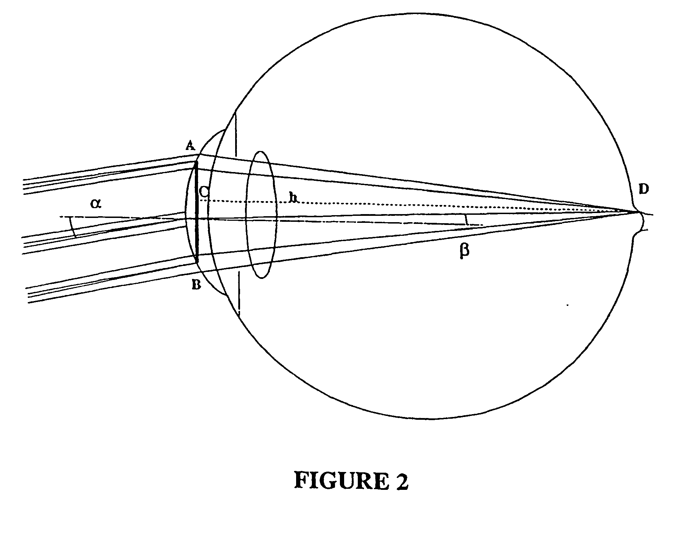

Referring to Figure 2, a preferred procedure of the present invention utilises a beam consisting of two sub-components with a fixed path difference. The nodal point of the scanning pattern is located at the cornea at position A. Lateral scanning takes place and a standard ophthalmoscopic image is recorded. Simultaneously, the points and/or areas where interference occurs are marked in a different colour on the same image. In doing so, the classification of these features which cause the interference is facilitated. The image is stored and the nodal point of the scanning pattern is moved to position B. Again, lateral scanning takes place and the points and/or areas of interference overlaid onto a standard ophthalmoscopic image are recorded. After storing the image the path difference between the sub-components of the illumination beam is changed by a certain amount (step width) and images at the positions A and B are acquired in the same manner as described above. The process of changing the path difference between the sub-components of the illumination beam and the acquisition of images at the positions A and B continues until the full three dimensional region of interest of the fundus of the eye is covered. in order to determine the distance of the feature D from the reference line AB computer software is used to detect those images in the stacks of images recorded from positions A and B respectively where the feature D is marked as causing interference. The path difference between the sub-components associated with these images is a measure of the distances AD and BD, respectively. The distance AB is controlled by the positions of certain optical elements of the apparatus shown in Figure 1 and therefore also known. As a result, the triangle ABD is completely determined without measuring any angles. Triangulation methods can now be used to calculate the length of any line, including the height h (the height of triangle ABD along CD) and the value of any angle of this triangle.

Further information such as the value of the refractive index can be obtained from angular measurements. The nodal point of the scanning pattern may be placed at position C on the cornea, halfway between positions A and B. The horizontal scan angle under which the feature D is imaged corresponds to a certain position of the horizontal scanner of the apparatus shown in Figure 1 and can be detected. The angle β, which describes the direction a beam has to travel from position C to reach feature D, can be calculated using trigonometric methods. The refractive index is then given as the quotient of sin and sin β.

Modification within the scope of the aforementioned invention may be readily effected by a person skilled in the art. Other alternative embodiments would involve the use of fibre-optic based light delivery systems including fibre-optic based components for beam splitting and re-combing and phase modulating. The position of the beam splitting, focussing and re-combining unit which is used to produce a reference beam focused on the cornea, may be changed within the lay-out of the complete set-up. The process of image and data acquisition may also be altered, for example, axial scanning may be carried out for a fixed lateral position or in conjunction with a line scan. It is to be understood, therefore, that this invention is not limited to the particular embodiments and methods described by way of example hereinabove, but by the appended claims.

## Claims

1. An ophthalmoscope for the three dimensional imaging of an eye including:
first beam modifying means for modifying an incident beam of short coherence length light to form a modified beam of first and second components having a mutual path difference and being capable of producing a detectable interference;
beam splitting means for splitting said modified beam into first and second beams;
second beam modifying means for changing the divergence of at least one of said first and second beams;
recombining means for thereafter recombining said first and second beams;
means for directing said recombined first and second beams towards a surface of said eye and scanning them across the surface; and
means for monitoring the first and second beams after reflection and detecting interference of the reflected first and second beams.

2. An imaging apparatus according to claim 1 further including steering means to vary a nodal point of the scanned first and second beams.

3. An imaging apparatus according to claim 1 or 2, wherein said first beam after said changing of the divergence of at least one of said first and second beams is focused on a position in front of said surface for reflection at said position.

4. An imaging apparatus according to claim 3 wherein said second beam is a collimated beam at said scanning means, for being focussed onto said surface.

5. An imaging apparatus according to any preceding claim wherein said scanning means is arranged for scanning in at least two dimensions.

6. An imaging apparatus according to any preceding claim, further including one or more sources of said incident beam of short coherence length light.

7. An imaging apparatus according to any preceding claim wherein said first beam modifying means includes means to modulate the phase difference between said first and second components.

8. An imaging apparatus according to any preceding claim wherein said first beam modifying means includes means to polarize said first and second components.

9. An imaging apparatus according to any preceding claim wherein said first beam modifying means includes interferometric means having at least one optical arm with adjustable mirror means.

10. An imaging apparatus according to any preceding claim, further including beamsplitting means for deflecting said reflected first and second beams to said monitoring and detecting means, when said reflected beams are returned along the optical path of the incident recombined first and second beams.

11. An imaging apparatus according to any preceding claim wherein said second beam modifying means includes beam focusing means.

12. An imaging apparatus according to any preceding claim, including means for varying the direction of incidence of said recombined beam towards said surface, whereby to obtain at said monitoring and detecting means alternate left and right images of substantially overlapping areas.

13. An imaging apparatus according to any preceding claim, for imaging and/or measuring a surface comprising an ocular fundus.

14. An imaging apparatus according to claim 13 wherein said incident beam is a laser beam, and the apparatus functions in use as a scanning laser ophthalmoscope.

15. An imaging apparatus according to claim 13 or 14 wherein said first and second beams are respectively a focussed beam arranged to be at least partially reflected from the cornea of an eye and a collimated beam for being focussed by the eye onto its fundus for reflection thereby.

16. An imaging apparatus according to any preceding claim, further including image analysing means to obtain three-dimensional topological data of said surface.

17. A method for the three dimensional imaging of an eye including:
modifying an incident beam of short coherence length light to form a modified beam of first and second components having a mutual path difference and being capable of producing a detectable interference;
splitting said modified beam into first and second beams;
changing the divergence of at least one of said first and second beams and thereafter recombining said first and second beams;
directing said recombined first and second beams towards a surface of said eye and scanning them across the surface; and
monitoring the first and second beams after reflection and detecting interference of the reflected first and second beams.

18. A method according to claim 17 further including varying a nodal point of the scanned first and second beams.

19. A method according to claim 17 or 18, wherein said first beam after said i changing of the divergence of at least one of said first and second beams is focussed on a position in front of said surface for reflection at said position.

20. A method according to claim 19 wherein said scanned second beam is a collimated beam for being focussed onto said surface.

21. A method according to any one of claims 17 to 20 wherein said scanning is in at least two dimensions.

22. A method according to any one of claims 17 to 21 wherein said modifying of said incident beam includes modulating the phase difference between said first and second components.

23. A method according to any one of claims 17 to 22 wherein said modifying of said incident beam includes polarizing said first and second components.

24. A method according to any one of claims 17 to 23 further including deflecting said reflected first and second beams for said monitoring and detecting, when said reflected beams are returned along the optical path of the incident recombined first and second beams.

25. A method according to any one of claims 17 to 24, including varying the direction of incidence of said recombined beam towards said surface, whereby to obtain for said monitoring and detecting alternate left and right images of substantially overlapping areas.

26. A method according to any one of claims 17 to 25, wherein said surface is an ocular fundus.

27. A method according to claim 26 wherein said incident beam is a laser beam and said method includes scanning laser ophthalmoscopy.

28. A method according to claim 26 or 27, wherein said first and second beams are respectively a focussed beam arranged to be at least partially reflected from the cornea of an eye, and a collimated beam for being focussed by the eye onto its fundus for reflection thereby.

29. A method according to any one of claims 17 to 28, further including obtaining three-dimensional topological data of said surface.

30. A method as claimed in claim 17, wherein:
said modifying of said incident beam includes
polarising said beam; and
modulating at least one of said sub components;
said scanning of said recombined beam includes
scanning said sub-components in a first direction; and
scanning said sub-components in a second direction different from said first direction;
and the method further includes the steps of
directing said sub-components through a beam steerer to provide a triangulation base by impinging said sub-components onto said surface from two different positions;
reflecting said sub-components onto said surface
whereby reflected light from said surface traverses au output path identical at least in part to said input path, including splitting said sub-components and directing a portion of the split sub-components through an aperture means towards detecting means coupled to signal processing means and display means, whereby the resultat image can be viewed and three dimensional topological data of said surface can be obtained.

31. A method of measuring the distance of a point D on a surface of the eye to a plane AB using an ophthalmoscope as described in claim 1, the ophthalmoscope including a beam steering means for directing the first and second components from point A and remote point B, the method comprising the following steps:
obtaining a first set of images of the surface of the eye, taken at a certain retinal depth from point A, at least one image in the first set of images including point D;
obtaining a second set of images of the surface of the eye, taken at a certain retinal depth from point B, at least one image in the second set of images including point D;
determining the distance AD from the path difference between the first and second beams of the ophthalmoscope associated with an image including point D in the first set of images;
determining the distance BD from the path difference between the first and second beams of the ophthalmoscope associated with an image including point D in the second set of images;
obtaining the distance AB from the position of predetermined optical elements of the ophthalmoscope; and
calculating the perpendicular distance from plane AB to point D using triangulation methods.

32. A method of measuring the distance of a point D on a surface of the eye to a plane AB according to claim 31, where the steps of obtaining a first and second set of images are obtained by alternately tahing images from point A and point B.

## Patentansprüche

1. Ophthalmoskop für das dreidimensionale Abbilden eines Auges, aufweisend
erste Strahlmodifiziermittel zum Modifizieren eines einfallenden Lichtstrahls von kurzer Kohärenzlänge in einen modifizierten Lichtstrahl mit ersten und zweiten Komponenten, die eine gegenseitige Wegdifferenz haben und zur Erzeugung einer detektierbaren Interferenz geeignet sind;
Strahlteilermittel zum Teilen des modifizierten Lichtstrahls in erste und zweite Lichtstrahlen,
zweite Strahlmodifiziermittel zum Ändern der Divergenz von mindestens einem der ersten und der zweiten Lichtstrahlen;
Zusammenführungsmittel zum anschließenden Zusammenführen der ersten und zweiten Lichtstrahlen;
Mittel zum Richten der zusammengeführten ersten und zweiten Lichtstrahlen auf eine Fläche des Auges und zum Abtasten der zusammengeführten Lichtstrahlen über die Fläche; und
Mittel zum Überwachen der ersten und zweiten Lichtstrahlen nach einer Reflektion und zum Detektieren einer Interferenz der reflektierten ersten und zweiten Lichtstrahlen.

2. Abbildungsgerät nach Anspruch 1 mit Lenkmitteln zum Verändern eines Knotenpunktes der abtastenden ersten und zweiten Lichtstrahlen.

3. Abbildungsgerät nach Anspruch 1 oder 2, bei dem der erste Lichtstrahl nach dem Ändern der Divergenz von mindestens einem der ersten und der zweiten Lichtstrahlen auf eine Position vor der genannten Fläche zur Reflektion an dieser Position fokussiert wird.

4. Abbildungsgerät nach Anspruch 3, bei dem der zweite Lichtstrahl an den genannten Abtastmitteln ein kollimierter Lichtstrahl ist, um auf die genannte Fläche fokussiert zu werden.

5. Abbildungsgerät nach einem der vorangehenden Ansprüche, bei dem die Abtastmittel zum Abtasten in mindestens zwei Dimensionen angeordnet sind.

6. Abbildungsgerät nach einem der vorangehenden Ansprüche mit einer oder mehr Quellen des genannten einfallenden Lichtstrahls von kurzer Kohärenzlänge.

7. Abbildungsgerät nach einem der vorangehenden Ansprüche, bei dem die ersten Strahlmodifiziermittel Mittel zum Modulieren der Phasendifferenz zwischen den ersten und zweiten Komponenten enthalten.

8. Abbildungsgerät nach einem der vorangehenden Ansprüche, bei dem die ersten Strahlmodifiziermittel Mittel zum Polarisieren der ersten und zweiten Komponenten enthalten.

9. Abbildungsgerät nach einem der vorangehenden Ansprüche, bei dem die ersten Strahlmodifiziermittel interferometrische Mittel mit mindestens einem optischen Arm mit verstellbaren Spiegelmitteln aufweisen.

10. Abbildungsgerät nach einem der vorangehenden Ansprüche weiter enthaltend Strahlteilermittel zum Ablenken der reflektierten ersten und zweiten Lichtstrahlen auf die Überwachungs- und Detektiermittel, wenn die reflektierten Lichtstrahlen entlang des Lichtweges der einfallenden zusammengeführten ersten und zweiten Lichtstrahlen zurückgelangen.

11. Abbildungsgerät nach einem der vorangehenden Ansprüche, bei dem die zweiten Strahlmodifiziermittel Mittel zum Fokussieren des Lichtstrahls aufweisen.

12. Abbildungsgerät nach einem der vorangehenden Ansprüche mit Mitteln zum Ändern der Einfallrichtung des zusammengeführten Lichtstrahls in Richtung auf die genannte Fläche, um an den Überwachungs- und Detektiermitteln alternierende linke und rechte Bilder von im Wesentlichen überlappenden Bereichen zu erhalten.

13. Abbildungsgerät nach einem der vorangehenden Ansprüche zum Abbilden und/oder zum Ausmessen einer einen Augenhintergrund enthaltenden Fläche.

14. Abbildungsgerät nach Anspruch 13, bei dem der einfallende Lichtstrahl ein Laserstrahl ist und das Gerät im Gebrauch als abtastendes Laserophtalmoskop arbeitet.

15. Abbildungsgerät nach Anspruch 13 oder 14, bei dem der erste Lichtstrahl ein fokussierter Lichtstrahl ist, der zumindest teilweise von der Hornhaut eines Auges reflektiert wird, während der zweite Lichtstrahl ein kollimierter Lichtstrahl ist, der vom Auge auf den Augenhintergrund zur Reflektion durch diesen fokussiert ist.

16. Abbildungsgerät nach einem der vorangehenden Ansprüche weiterenthaltend Bildanalysiermittel zum Erhalten von dreidimensionalen topologischen Daten der genannten Fläche.

17. Verfahren zum dreidimensionalen Abbilden eines Auges,
bei dem ein einfallender Lichtstrahl von kurzer Kohärenzlänge in einen modifizierten Lichtstrahl mit ersten und zweiten Komponenten modifiziert wird, bei dem die Komponenten eine gegenseitige Wegdifferenz aufweisen und zum Erzeugen einer detektierbaren Interferenz geeignet sind;
bei dem der modifizierte Lichtstrahl in erste und zweite Lichtstrahlen geteilt wird;
bei dem die Divergenz von mindestens einem der ersten und zweiten Lichtstrahlen verändert wird und anschließend der erste und zweite Lichtstrahl zusammenangeführt werden;
bei dem die zusammengeführten ersten und zweiten Lichtstrahlen auf eine Fläche des Auges gerichtet und über die Fläche abgetastet werden; und
bei dem die ersten und zweiten Lichtstrahlen nach der Reflektion überwacht und eine Interferenz der reflektierten ersten und zweiten Lichtstrahlen detektiert wird.

18. Verfahren nach Anspruch 17, bei dem ein Knotenpunkt der abtastenden ersten und zweiten Lichtstrahlen verändert wird.

19. Verfahren nach Anspruch 17 oder 18, bei dem der erste Lichtstrahl nach dem Ändern der Divergenz von mindestens einem der ersten und zweiten Lichtstrahlen auf eine Position vor der Fläche zur Reflexion an dieser Position fokussiert wird.

20. Verfahren nach Anspruch 19, bei dem der abtastende zweite Lichtstrahl ein kollimierter Lichtstrahl ist um auf die genannte Fläche fokussiert zu werden.

21. Verfahren nach einem der Ansprüche 17 - 20, bei dem das Abtasten in mindestens zwei Dimensionen vorgenommen wird.

22. Verfahren nach einem der Ansprüche 17 - 21, bei dem das Modifizieren des einfallenden Lichtstrahls ein Modulatieren der Phasendifferenz zwischen den ersten und zweiten Komponenten beinhaltet.

23. Verfahren nach einem der Ansprüche 17 - 22, bei dem das Modifizieren des einfallenden Lichtstrahls das Polarisieren der ersten und zweiten Komponenten beinhaltet.

24. Verfahren nach einem der Anspruche 17 - 23, bei dem die reflektierten ersten und zweiten Lichtstrahlen für das Überwachen und Detektieren abgelenkt werden, wenn die reflektierten Lichtstrahlen entlang des Lichtweges der einfallenden, zusammengeführten ersten und zweiten Lichtstrahlen zurückgelangen.

25. Verfahren nach einem der Ansprüche 17 - 24, bei dem der Einfallsrichtung des zusammengeführten Lichtstrahls in Richtung auf die genannte Fläche verändert wird, um für das Überwachen und Detektieren alternierende linke und rechte Bilder mit im Wesentlichen überlappenden Bereichen zu erhalten.

26. Verfahren nach einem der Ansprüche 17 - 25, bei dem die genannte Fläche ein Augenhintergrund ist.

27. Verfahren nach Anspruch 26, bei dem der einfallende Lichtstrahl ein Laserstrahl ist und das Verfahren eine abtastende Laserophthalmoskopie enthält.

28. Verfahren nach Anspruch 26 oder 27, bei dem der erste Lichtstrahl ein fokussierter Lichtstrahl ist, der so angeordnet ist, dass er zumindest teilweise von der Hornhaut eines Auges reflektiert wird, und bei dem der zweite Lichtstrahl ein kollimierter Lichtstrahl zur Fokussierung durch das Auge auf den Augenhintergrund zur Reflektion durch diesen ist.

29. Verfahren nach der Ansprüche 17 - 28, bei dem dreidimensionale topologische Daten der Fläche erhalten werden.

30. Verfahren nach Anspruch 17,
bei dem das Modifizieren des einfallenden Lichtstrahls
ein Polarisieren des Lichtstrahls; und
das Modulieren von mindestens einer der Subkomponenten aufweist;
bei dem das Abtasten des zusammengeführten Lichtstrahls
ein Abtasten der genannten Subkomponenten in einer ersten Richtung;
und ein Abtasten der genannten Subkomponenten in einer von der ersten Richtung unterschiedlichen zweiten Richtung umfasst;
und wobei das Verfahren folgendene Schritte umfasst
Richten der Subkomponenten mittels einer Strahlleuchtung, um durch Auftreffen der Subkomponenten auf die genannte Fläche aus zwei unterschiedlichen Positionen eine Triangulationsbasis zu erhalten;
Reflektieren der genannten Subkomponenten auf die Fläche;
wodurch reflektiertes Licht von der genannten Fläche einen Ausgangspfad durchläuft, der mindestens teilweise identisch mit dem Eingangspfad ist, und die genannten Subkomponenten geteilt und ein Anteil der geteilten Subkomponenten durch Blendenmittel auf die Detektiermittel gerichtet werden, die ihrerseits an Signalverarbeitungs- und Anzeigemittel gekoppelt sind, wodurch das resultierende Bild betrachtet und die dreidimensionalen topologischen Daten der Fläche erhalten werden.

31. Verfahren zum Messen des Abstandes eines Punktes D auf einer Fläche des Auges von einer Ebene AB unter Verwendung eines Ophthalmoskops gemäß Anspruch 1, bei dem das Ophthalmoskop Strahlleuchtungsmittel zum Richten der ersten und zweiten Komponenten vom Punkt A und dem entfernten Punkt B aufweist, und wobei das Verfahren folgende weitere Schritte umfasst;
Erhalten eines ersten Satzes von Abbildungen der Fläche des Auges, der in einem bestimmten retinalen Abstand vom Punkt A abgenommen wird, wobei mindestens eine Abbildung des ersten Satzes von Abbildungen dem Punkt D enthält;
Erhalten eines zweiten Satzes von Abbildungen der Fläche des Auges, der in einem bestimmten retinalen Abstand vom Punkt B abgenommen wird und mindestens eine Abbildung aufweist, die den Punkt D umfasst;
Bestimmen des Abstandes AD aus der Bilddifferenz der ersten und zweiten Lichtstrahlen des Ophthalmoskop, die mit dem in Punkt D im ersten Satz der Abbildungen enthaltenden Bild verknüpft sind;
Bestimmen des Abstandes BD aus der Wegdifferenz zwischen den ersten und zweiten Lichtstrahlen des Ophthalmoskop, die mit dem in Punkt D im zweiten Satz der Abbildungen enthaltenen Bild verknüpft sind;
Erhalten des Abstandes AB aus der Position vorbestimmter optischer Elemente des Ophthalmoskops;
Berechnen des lotrechten Abstandes zwischen der Ebene AB und dem Punkt D unter Verwendung von Triangulationsverfahren.

32. Verfahren zum Messen des Abstandes von einem Punkt D auf einer Fläche des Auges zu einer Ebene AB gemäß Anspruch 31, bei dem die Schritte des Erhaltens des ersten und zweiten Satzes von Abbildungen durch alternierendes Abnehmen von Abbildungen vom Punkt A und Punkt B erhalten werden.

## Revendications

1. un ophtalmoscope, pour l'imagerie tridimensionnelle d'un oeil, comprenant :
des premiers moyens de modification de faisceau, pour modifier un faisceau incident, à lumière à courte longueur de cohérence, pour former un faisceau modifié de premier et deuxième composants, ayant une différence de chemins mutuels et étant capables de produire une interférence détectable ;
des moyens de division de faisceau, pour diviser ledit faisceau modifié en des premier et deuxième faisceaux ;
des deuxièmes moyens de modification de faisceaux pour modifier la divergence d'au moins l'un desdits premier et deuxième faisceaux :
des moyens de recombinaison pour ensuite recombiner lesdits premier et deuxième faisceaux ;
des moyens pour diriger lesdits premier et deuxième faisceaux recombinés vers une surface dudit oeil et les faire passer en exploration sur la surface ; et
des moyens, pour surveiller les premier et deuxième faisceaux après réflexion et détecter une interférence des premier et deuxième faisceaux réfléchis.

2. Un dispositif d'imagerie selon la revendication 1, comprenant en outre des moyens de guidage directionnels, pour faire varier un point nodal des premier et deuxième faisceaux effectuant un balayage d'exploration.

3. Un dispositif d'imagerie selon la revendication 1 ou 2, dans lequel ledit premier faisceau, après ladite modification de la divergence d'au moins l'un desdits premier et deuxième faisceaux, est focalisé à une position à l'avant de ladite surface pour réflexion à ladite position.

4. Un dispositif d'imagerie selon la revendication 3, dans lequel ledit deuxième faisceau est un faisceau colimaté au niveau desdits moyens de balayage d'exploration, pour être focalisé sur ladite surface.

5. Un dispositif d'imagerie selon l'une quelconque des revendications précédentes, dans lequel lesdits moyens de balayage d'exploration sont agencés pour explorer en au moins deux dimensions.

6. Un dispositif d'imagerie selon l'une quelconque des revendications précédentes, comprenant en outre une ou plusieurs sources dudit faisceau incident de lumière à courte longueur de cohérence.

7. Un dispositif d'imagerie selon l'une quelconque des revendications précédentes, dans lequel lesdits premiers moyens de modification de faisceau comprennent des moyens pour moduler la différence de phase entre lesdits premier et deuxième composants.

8. Un dispositif d'imagerie selon l'une quelconque des revendications précédentes, dans lequel lesdits premiers moyens de modification de faisceau comprennent des moyens pour polariser lesdits premier et deuxième composants.

9. Un dispositif d'imagerie selon l'une quelconque des revendications précédentes, dans lequel lesdits premiers moyens de modification de faisceau comprennent des moyens interférométriques, ayant au moins un bras optique ayant des moyens de miroirs ajustables.

10. Un dispositif d'imagerie selon l'une quelconque des revendications précédentes, comprenant en outre des moyens de division de faisceau pour dévier lesdits premier et deuxième faisceaux réfléchis vers lesdits moyens de surveillance et de détection, lorsque lesdits faisceaux réfléchis sont retournés le long du chemin optique desdits premier et deuxième faisceau recombinés incidents.

11. Un dispositif d'imagerie selon l'une quelconque des revendications précédentes, dans lequel lesdits moyens de modification de faisceau comprennent des moyens de focalisation de faisceaux.

12. Un dispositif d'imagerie selon l'une quelconque des revendications précédentes, comprenant des moyens pour faire varier la direction d'incidence dudit faisceau recombiné vers ladite surface, de manière à obtenir au niveau desdits moyens de surveillance et de détection des images alternées, gauche et droite, de zones sensiblement en chevauchement.

13. Un dispositif d'imagerie selon l'une quelconque des revendications précédentes, pour imager et/ou mesurer une surface comprenant un fond de l'oeil.

14. Un dispositif d'imagerie selon la revendication 13, dans lequel ledit faisceau incident est un faisceau laser et le dispositif en utilisation fonctionne comme un ophtalmoscope à laser à balayage.

15. Un dispositif d'imagerie selon la revendication 13 ou 14, dans lequel lesdits premier et deuxième faisceaux sont respectivement un faisceau focalisé, agencé pour être au moins en partie réfléchi par la cornée d'un oeil, et un faisceau colimaté, devant être focalisé par l'oeil sur son fond pour être réfléchi de cette manière.

16. Un dispositif d'imagerie selon l'une quelconque des revendications précédentes, comprenant en outre des moyens d'analyse d'image, pour obtenir des données topologiques tridimensionnelles de ladite surface.

17. Un procédé pour l'imagerie tridimensionnelle d'un oeil, comprenant :
la modification d'un faisceau incident, à lumière à courte longueur de cohérence, pour former un faisceau modifié de premier et de deuxième composants, ayant une différence de chemins mutuels et capables de produire une interférence détectable ;
la division dudit faisceau modifié en des premier et deuxième faisceaux ;
la modification de la divergence d'au moins l'un desdits premier et deuxième faisceaux et, ensuite, la recombinaison desdits premier et deuxième faisceaux ;
la direction desdits premier et deuxième faisceaux recombinés vers une surface dudit oeil et leur balayage d'exploration sur la surface ; et
la surveillance des premier et deuxième faisceaux, après réflexion et détection d'une interférence des premier et deuxième faisceaux réfléchis.

18. Un procédé selon la revendication 17, comprenant en outre la modification d'un point nodal des premier et deuxième faisceaux, ayant effectué un balayage d'exploration.

19. Un procédé selon la revendication 17 ou 18, dans lequel ledit premier faisceau, après ladite modification de la divergence d'au moins l'un desdits premier et deuxième faisceaux, est focalisé à une position située à l'avant de ladite surface, pour une réflexion à ladite position.

20. Un procédé selon la revendication 19, dans lequel ledit deuxième faisceau ayant subi un balayage d'exploration est un faisceau colimaté devant être focalisé sur ladite surface.

21. Un procédé selon l'une quelconque des revendications 17 à 20, dans lequel ledit balayage d'exploration se fait en au moins deux dimensions.

22. Un procédé selon l'une quelconque des revendications 17 à 21, dans lequel ladite modification dudit faisceau incident comprend la modulation de la différence de phase entre lesdits premier et deuxième composants.

23. Un procédé selon l'une quelconque des revendications 17 à 22, dans lequel ladite modification dudit faisceau incident comprend la polarisation desdits premier et deuxième composants.

24. Un procédé selon l'une quelconque des revendications 17 à 23, comprenant en outre la déviation desdits premier et deuxième faisceaux déviés pour ladite surveillance et détection, lorsque lesdits faisceaux réfléchis sont retournés le long du chemin optique des premier et deuxième faisceaux recombinés incidents.

25. Un procédé selon l'une quelconque des revendications 17 à 24, incluant la modification de la direction d'incidence dudit faisceau recombiné vers ladite surface, de manière à obtenir ladite surveillance et détection d'images alternées, gauche et droite, de zones sensiblement en chevauchement.

26. Un procédé selon l'une quelconque des revendications 17 à 25, dans lequel ladite surface est un fond de l'oeil.

27. Un procédé selon la revendication 26, dans lequel ledit faisceau incident est un faisceau laser et ledit procédé comprend une ophtalmoscopie par balayage au laser.

28. Un procédé selon la revendication 26 ou 27, dans lequel lesdits premier et deuxième faisceaux sont respectivement un faisceau focalisé, agencé pour être au moins partiellement réfléchi par la cornée de l'oeil, et un faisceau collimaté, devant être focalisé par l'oeil sur son fond pour être de cette manière réfléchi.

29. Un procédé selon l'une quelconque des revendication 17 à 28, comprenant en outre l'obtention de données topologiques tridimensionnelles de ladite surface.

30. Un procédé selon la revendication 17, dans lequel :
ladite modification dudit faisceau incident comprend
la polarisation dudit faisceau ; et
la modulation d'au moins l'un desdits sous-composants ;
ledit balayage d'exploration dudit faisceau recombiné comprend :
le balayage d'exploration desdits sous-composants dans une première direction ; et
le balayage d'exploration desdits sous-composants dans une deuxième direction, différente de ladite première direction ;
et le procédé comprend en outre les étapes de
direction desdits sous-composants à travers un directeur de faisceau, pour fournir une base de triangulation en faisant que lesdits sous-composants touchent ladite surface depuis deux positions différentes ;
réflexion desdits sous-composants sur ladite surface ;
de manière que la lumière réfléchie par ladite surface traverse un chemin de sortie identique au moins en partie audit chemin d'entrée, y compris division desdits sous-composants et direction d'une partie des sous-composants divisés à travers des moyens d'ouverture vers des moyens de détection couplés à des moyens de traitement de signal et à des moyens d'affichage, de manière que l'image résultante puisse être observée et que des données topologiques tridimensionnelles de ladite surface puissent être obtenues.

31. Un procédé de mesure de la distance d'un point D sur une surface de l'oeil vis-à-vis d'un plan AB, en utilisant un ophtalmoscope tel que décrit à la revendication 1, l'ophtalmoscope comprenant un moyen de guidage directionnel de faisceau pour diriger les premier et deuxième composants depuis le point A et le point B distant, le procédé comprenant les étapes suivantes :
obtention d'un premier jeu d'images de la surface de l'oeil, prises à une certaine profondeur rétinienne du point A, au moins une image du premier jeu d'images comprenant un point D ;
obtention d'un deuxième jeu d'images de la surface de l'oeil, prises à une certaine profondeur rétinienne du point B, au moins une image du deuxième jeu d'images comprenant un point D ;
détermination de la distance AD, à partir de la différence de chemin entre les premier et deuxième faisceaux de l'ophtalmoscope, associés à une image incluant un point D dans le premier jeu d'images ;
détermination de la distance BD, à partir de la différence de chemin entre les premier et deuxième faisceaux de l'ophtalmoscope, associés à une image incluant un point D dans le deuxième jeu d'images ;
obtention de la distance AB à partir de la position d'éléments optiques prédéterminés de l'ophtalmoscope ; et
calcul de la distance perpendiculaire vis-à-vis du plan AB au point D, en utilisant des procédés de triangulation.

32. Un procédé de mesure de la distance d'un point D sur une surface de l'oeil à un plan AB selon la revendication 31, dans lequel les étapes d'obtention, d'un premier et d'une deuxième jeux d'images, sont obtenues par prises alternées d'images depuis le point A et le point B.
